# EUROPEAN PATENT SPECIFICATION

(11) **EP 1 683 541 B1**
(45) Date of publication and mention of the grant of the patent: **25.04.2012**
(21) Application number: 06250349.5
(22) Date of filing: 24.01.2006
(51) Int. Cl.: A61M 25/10

(54) **Balloon catheter for use in positioning a stent in a curved segment of a blood vesel**
Ballonkatheter für Stentpositionierung in einem Blutgefässkurvensegment
Cathéter à ballon utilisable pour positioner un stent dans une partie courbe d'un vaisseau sanguin

(30) Priority: 24.01.2005 US 905863; 24.01.2005 US 905864
(43) Date of publication of application: 26.07.2006
(73) Proprietor: Ebeid, Makram R., Ridgeland, MS 39157 (US)
(72) Inventor: Ebeid, Makram R., Ridgeland, MS 39157 (US)
(74) Representative: Fox-Male, Nicholas Vincent Humbert

(56) References cited:
- US-A- 5 116 305
- US-A- 5 814 016
- US-A1- 2001 016 725

## Description

### BACKGROUND OF THE INVENTION

Field of the Invention. This invention relates in general to balloon catheters, and more particularly to a balloon catheter that can be used to perform balloon angioplasty or dilation of a narrowed curved vessel or to position a stent in a narrowed curved vessel such as the aortic arch located in the heart.

Prior Art. The most common balloons used to perform balloon angioplasty or to position a stent in a vessel have a straight profile. When such balloons are inflated across curved sections of a vessel, such as the aortic arch, they force a straightening of the vessel that carries the risk of perforation of the vessel wall. To avoid this risk extreme care must be taken when inserting a straight balloon in a curved segment of a vessel in order to avoid perforation of the vessel wall. This risk can increase when a metallic stent has been crimped or otherwise attached to the exterior surface of the balloon. To minimize such risk it is known to shorten the balloon length. However, such shortening may result in a balloon that does not adequately perform the angioplasty or dilation of the vessel or allow for the positioning of a desired stent where needed.

Additionally difficulties arise when one attempts to expand and position the stent in the curved segment of the vessel. In addition to increased risk of vessel wall perforation, another of the more common difficulties is the migration or slipping of the stent from the desired location because of its straight profile. This results in its movement away from the curved vessel section to settle along the straight section of the vessel. Over dilatation of the balloon have been utilized to overcome this stent movement. However, over dilation of the balloon increases the pressure of the balloon or attached stent against the vessel wall and increases the likelihood of vessel wall perforation.

It is known that to supplement the cardiac pumping action one can use an intra aortic balloon pump (IABPs) having a pre-curved balloon end to better enable the balloon to follow a curved arterial passage or to permit the balloon to remain stable when positioned in a curved arterial section during pumping. To position the balloon end a separate stiff straightening member is used to insert the IABP during the initial states of balloon insertion and then is removed once the balloon end is in position. However, in such balloon construction the cardiac (forward) end of the balloon is exposed to higher pressure than the opposite (tail) end of the balloon. This pressure differential may cause an undesirable folding in the tail end of the balloon during intra aortic balloon pumping. Such undesirable folding of the balloon reduces the lifetime and reliability and could eventually lead to premature failure. These balloons are designed to inflate and deflate quickly with each cardiac cycle and not to exert pressure on the vessel walls. This undesirable effect can by miumized by maintaining a slight tension on the balloon membrane in the inflated condition. This is achieved by utilizing a support member disposed within and extends from the catheter into the balloon where it terminates at the tip of the balloon that is proximal to the heart. This support member is adjacent to and in contact with the outer curve of the balloon membrane while the balloon is inflated. This is achieved by making the member of a length that is at least equal to that of the outer curve of the balloon wall. Such a complicated structure may be desirable for an IABP requiring constant rapid inflation and deflation of the balloon to achieve the desired pumping action and at the same time avoiding exerting pressure on the vessel wall. This makes these balloons unsuitable for angiplasty purposes that require the balloons to maintain high pressure on the vessel so as to enlarge the vessel or in essence cause controlled trauma to the vessel. Thus, the structure of the angioplasty balloons differs substantially than the aortic balloons, and the design previously described to curve the 1AB would be unsuitable for angioplasty purposes. Additionally, a more simple structure is desirable for balloon angioplasty, or dilation of a narrowed curved vessel, or to position a stent in a narrowed curved vessel such as the aortic arch.

None of these prior art combinations provide the desired simplicity, dependability, and risk minimization as is desired.

US Published Patent Specification 2001/016725 relates to a cardioplegia catheter with a multi-lumen arrangement designed to deliver a heart-arresting solution/medicine called cardioplegia while occluding the aorta. This document does not describe a balloon suitable for fixing to an angioplasty catheter for at least performing angioplasty.

### OBJECTS AND SUMMARY OF THE INVENTION

The present invention provides a balloon as defined in Claim 1.

The balloon may include the features of any one or more of dependent Claims 2 to 4.

The present invention also provides a balloon catheter as defined in Claim , 5.

The balloon catheter may include the feature of any one of more of dependent Claims 6 to 14.

Therefore, one object of this invention is to provide an improved balloon catheter that reduces the risk of the perforation of the aortic arch or other curved vessel wall during balloon dilating the vessel.

Another object of this invention is to provide an improved catheter balloon that reduces the risk of the perforation of the aortic arch or other curved vessel wall when a stent is being forced along the curved section of the vessel wall during implantation or enlargement of the stent after it has been positioned.

Another object of this invention is to provide an improved catheter balloon that shapes the stent to better conform to the curved segment of the vessel where the stent is to be implanted.

Another object of this invention is to provide an improved catheter balloon that reduces the migration of the stent is the aortic arch or other similar curved vessel.

Still another object of this invention is to provide an improved catheter balloon that minimizes the risk of vessel wall rupture perforation of the aorta arch or curved segment wall of a similar vessel.

Other objects and advantages of this invention shall become apparent from the ensuing descriptions of the invention.

Accordingly, in one embodiment an improved balloon catheter is utilized wherein the balloon is mounted in an off-center fashion on the proximal end of the catheter shaft; i.e., the entry and exit points of the shaft through the balloon are not in the center axis of the balloon cylinder. It is preferred that the balloon is mounted so that the vertical distance from the shaft to the upper wall surface of the balloon is greater than the vertical distance from the shaft to the lower wall surface of the balloon when the balloon is inflated. In this embodiment the balloon is generally of a cylindrical tubular shape, but with tapering at its opposite ends to enforce the pre-curved configuration of the proximal end. In this configuration the exterior surface of the upper wall surface will become generally convex following the shaft curve while the exterior lower wall surface will become generally concave. The degree of curvature can be controlled by the degree the entry and exit points are positioned off-center from the generally cylindrical center section of the balloon. In another preferred embodiment the catheter is constructed having a pre-curved segment at its proximal end to which the balloon is attached to facilitate curving the balloon with or without a stent crimped or otherwise attached to the balloon.

In another preferred embodiment the generally cylindrical center section of the balloon is molded having a pre-curved shape when inflated, and more preferably the generally curved shape is about that of the pre-curved shaft segment extending through the interior cavity of the balloon.

In another embodiment the balloon catheter includes two separate balloons attached to the catheter. In this embodiment one balloon is longer than the other balloon. The longer balloon is affixed to the convex side of the outer surface of the proximal end of the catheter shaft. The shorter balloon is affixed to the concave side of the outer surface of the distal end in a manner that will cause the two balloons to oppose one another when inflated. The shorter balloon is tapered from its center section to its opposing ends and when fully inflated will have less volume than the longer balloon when it is fully inflated. In a preferred embodiment the shorter balloon is affixed to the shaft in a manner to permit fluid to fill and expand the balloon from the center section of the balloon and then to the opposing ends of the balloon. The shorter balloon is inflated first to contact the lower convex section of the interior surface of the curved vessel wall and act as the supporting fulcrum for the larger balloon. Due to the structure of the shorter balloon the diameter of the center section of the balloon will be greater than the decreasing diameters of its opposing end sections. Because the opposing ends and the center of the shorter balloon are affixed to the shaft the inflation of the smaller balloon will cause the distal section of the shaft to become more curved. In this manner the degree of shaft curvature can be controlled by the amount of inflation of the shorter balloon. In a preferred embodiment the longer balloon is constructed so that on inflation the degree of tapering of its opposing ends is less than that of the shorter balloon. It is also preferred that the longer balloon be filled in a manner that the fluid is introduced in the balloon end sections and then fills the center section of the balloon. In another preferred embodiment the opposing end sections of the longer balloon will have greater elasticity than the center section to permit their expansion more rapidly than the center section. It is more preferred that the degree of elasticity be set to permit the end sections to contact the interior surface of the vessel wall before the center section. This will also permit greater control in obtaining the desired curvature of the shaft that is also controlled by the degree of inflation of the smaller balloon.

In another embodiment in the one balloon assembly described above an expandable stent is mounted about the balloon. In the two balloon assembly described above an expandable stent is mounted about both balloons. In both cases the expansion of the balloons will cause the stent to expand until it contacts the interior surfaces of the vessel wall. In a preferred embodiment the stent is constructed to form a curved outer surface that will generally conform to the curvature of the curved vessel wall. In operation once the stent is in place the balloons are deflated in a conventional manner by withdrawing the fluid inside the balloons and then removing the catheter with the balloons attached from the vessel.

### BRIEF DESCRIPTION OF THE DRAWINGS

The accompanying drawings illustrate a preferred embodiment of this invention. However, it is to be understood that these embodiments are not intended to be exhaustive, nor limiting of the invention. They are but examples of some of the forms in which the invention may be practiced.
Figure 1 illustrates a balloon catheter to which is attached a balloon folded in position for insertion into an artery, and with a metallic stent crimped about the balloon.
Figure 2A is a side view of a typical inflated straight balloon with its central axis illustrated by the dashed line.
Figure 2B is a side view of an inflated curved balloon of this invention with its central axis illustrated by the dashed line.
Figure 3 is a side view of a preferred embodiment of the curved balloon of Figure 2B to which has been attached to the catheter.
Figure 4 is an enlargement of insert 4 of Figure 3 illustrating the attachment of the catheter to the proximal end of the balloon.
Figure 5 shows an embodiment not forming part of the invention in a cross-sectional view taken along lines 5-5 of Figure 3.
Figures 6A-6C illustrate the progressive steps of inserting and inflating a preferred embodiment of the balloon across the aortic arch of the human heart.
Figure 7 is a cutaway perspective view of one preferred embodiment of the two-balloon assembly of this invention.
Figure 8 is a horizontal cross-sectional view taken along lines 8-8 of Figure 7.
Figure 9 is a cross-sectional view taken along lines 9-9 of Figure 7.
Figure 10 is a cross-sectional view taken along lines 10-10 of Figure 7.
Figure 11 is a cross-sectional view taken along lines 11-11 of Figure 7.
Figure 12 is a vertical cross-sectional view of insert 12 of Figure 7.

### PREFERRED EMBODIMENTS OF THE INVENTION

Without any intent to limit the scope of this invention, reference is made to the figures in describing the preferred embodiments of the invention.

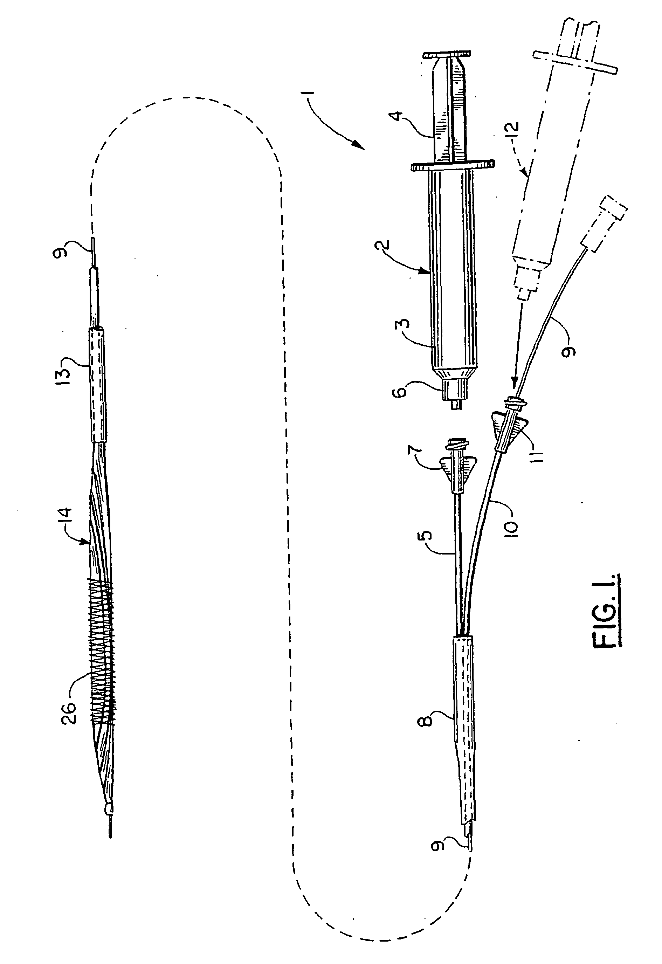

Referring to Figure 1 a preferred embodiment of balloon catheter **1** is illustrated. Catheter **1** includes a syringe **2** having a barrel **3** constructed to contain a sterile fluid and a plunger **4** slidingly fitting into barrel **3** to force the sterile fluid through elongated tubing **5** that is operatively attached to the nozzle **6** by a conventional connector **7.** Catheter **1** also includes a second elongated support tubing **8** sized to permit tubing **5** to be inserted into support tubing **8.** It also includes a flexible guide wire **9** sized to be inserted into and through support tubing **8.** In a preferred embodiment wire **9** is first inserted into a third elongated tubing **10** which in turn is sized to be inserted into support tubing **8** along with second tubing **5.** In this preferred embodiment tubing **10** is provided with a conventional connecting member **11** that can be used to attached to a second syringe **12** that can be used to withdraw fluids from the aortic vessel.

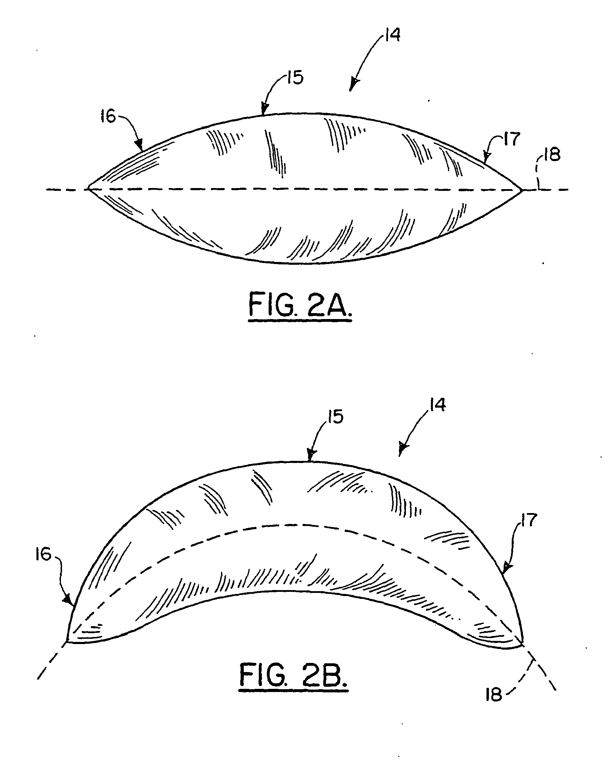

Attached to the proximal end **13** of catheter **1** is balloon **14.** As best seen in Figures 2A and 2B, balloon **14** has when inflated a center section 15 with opposing end sections **16** and **17,** respectively, that are tapered about a central longitudinal axis **18** running though the middle of the vertical cross-sections formed by balloon **14.** In the preferred embodiment illustrated in Figure 2B balloon **14** is constructed to be naturally curved when inflated.

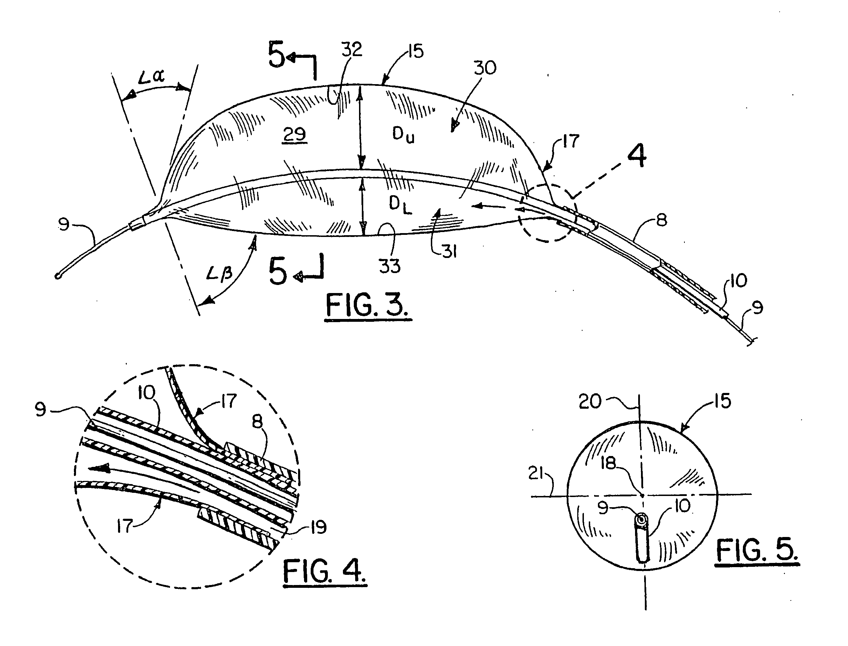

Referring now to Figures 3-5, support tubing **8** is inserted through a balloon opening **19** located in tapered end section **17** and sealing attached thereto. Balloon opening **19** is not located on axis **18,** but at a position on the vertical center line **20** of the cross-section of the balloon taken at opening **19,** and below the horizontal line **21** as illustrated in Figure 5. However, in the invention, opening 19 is positioned both below the horizontal line 21 and off of vertical axis 20.

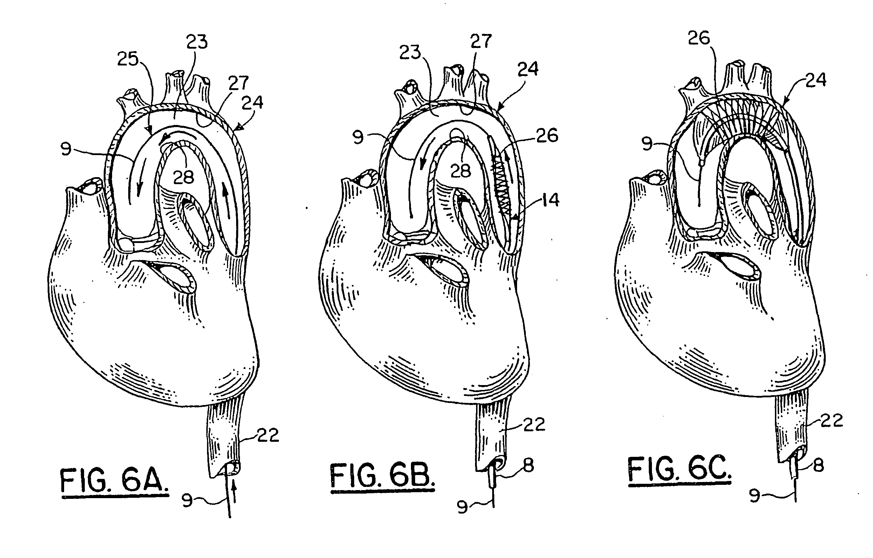

As illustrated in Figures 6A - 6C, guide wire **9** is inserted through artery **22,** and then across aortic arch **23** of the aorta **24.** It is preferred that guide wire **9** have a proximal section **25** shaped with at least some curvature to facilitate movement through the curved sections of the artery **22** and the aorta arch **23.** With wire **9** in place, support tubing **8** with attached balloon **14** and stent **26** is slide over wire **9** and into the aorta **24** as illustrated in Figure 6B. Balloon **14** and stent **26** are then positioned across aortic arch **23.** Once positioned balloon **14** is inflated to expand stent **26** until it contacts, supports and separates the opposing aortic arch inner wall linings **27** and **28**, respectively, the desired distance as illustrated in Figure 6C. Once stent **26** has been expanded, balloon **14** is deflated by pulling back plunger **4** to withdraw the sterile fluid back into syringe barrel 3. Catheter **1**, along with guide wire **9** are then extracted from the patient leaving stent 26 in position.

Due to the off-center tubing insertion positions as seen in Figure 3, balloon cavity **29** is made up of an upper section **30** and a lower section **31**. The distance **D_{U}** from support tubing **8** and the interior wall surface **32** forming upper cavity section **30** is greater than the distance **D_{L}** from support tubing **8** and the interior wall surface **33.** Additionally, the length of interior wall surface **32** will also be greater than the length of interior wall surface 33. Thus, as cavity **29** is inflated by filling it with the sterile fluid, the desired curvature of support tubing **8** can be achieved. By selecting the insertion positions of support tubing **8,** the degree of inflation, and the elasticity of wall surfaces **32** and **33,** one can control angle **α** and angle **β** to achieve the desired support tubing curvature.

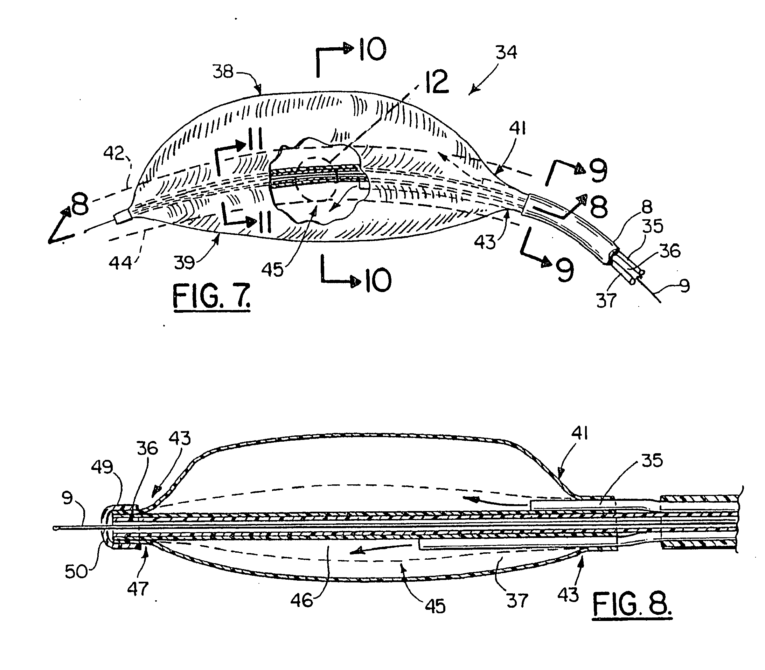

In an alternate embodiment illustrated in Figures 7-12, a two-balloon catheter **34** is utilized. In this embodiment three flexible elongated tubings **35, 36**, and **37**, respectively, are inserted through support tubing **8.** Tubings **35** and **37** are operatively attached to separate syringes (not shown) in similar fashion to that illustrated in Figure 1 to pennit sterile fluid to be inserted into balloons **38** and **39**, respectively. Guide wire **9** is insertable through tubing **36.**

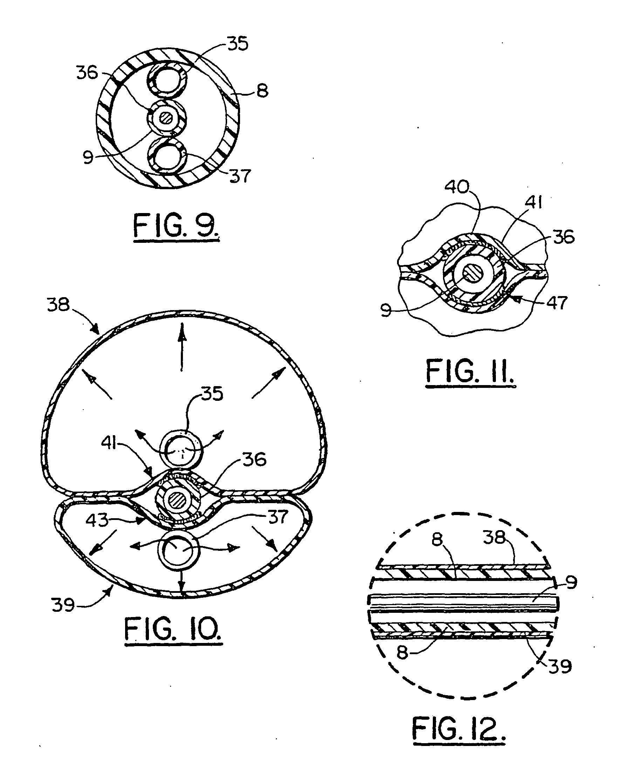

As best illustrated in Figure 10, upper balloon **38** is sealing attached by conventional means, such as glue **40**, at its distal end **41** to tubing **36**. It is preferred that tubing **36** be inserted through upper balloon **38** below its central longitudinal axis **42**. Lower balloon **39** is attached at its distal end **43** to tubing **36.** However, it is preferred that tubing **36** be inserted through lower balloon **39** above its central longitudinal axis **44**. In a more preferred embodiment tubing **36** extends into the longitudinal center section **45** of balloon cavity **46** of lower balloon **39** to better ensure uniform distribution of the sterile fluid during the initial stages of inflation of balloon **39.** The proximal end **47** of lower balloon **39** is affixed to tubing **36**. In similar fashion the proximal end **48** of upper balloon **38** is also affixed to tubing **36.** In a preferred embodiment cap **49** is fitted over both proximal ends **47** and **48,** respectively, to better ensure the affixing to tubing **36.** Cap **49** is provided with an opening **50** through which guide wire **9** can pass.

In operation once balloon catheter **34** is in position across aortic arch **23**, lower balloon **39** is first partially inflated by the insertion of sterile fluid through tubing **37**. Because lower balloon **39** is shorter than upper balloon **38** and when inflated has less volume than upper balloon **38,** lower balloon **39** can be utilized to obtain the desired curvature across the aortic arch by the proper degree of bending of guide wire **9.** To decrease the likelihood that balloon **39** might rotate about guide wire **9**, balloon **39** is only partially inflated, then upper balloon **38** is partially inflated to prevent any rotation of balloon **39.** Balloon **39** is then further inflated. If needed, balloon **38** can be further inflated. This controlled alternate partially inflation of both balloons **38** and **39** better ensures that the balloons are properly shaped and positioned across the aortic arch **23.**

There are of course other alternate embodiments which are obvious from the foregoing descriptions of the invention which are intended to be included within the scope of the invention as defined by the following claims.

## Claims

1. A balloon (14, 38, 39) suitable for affixing to an angioplasty catheter (1, 34) having a pre-curved segment for positioning a stent (26) or performing angioplasty in a curved segment of a vessel and further for inflating the balloon (14, 38, 39), wherein the balloon (14, 38, 39) comprises:
an envelope having a middle elongated, generally tubular, inflatable section (15) and two end sections (16, 17), the two end sections (16, 17) being arranged to have respective sloped wall surfaces when the balloon (14, 38, 39) is affixed to a pre-curved segment of a catheter (1, 34), the envelope defining an interior cavity (29), the envelope also comprising a receiving opening (19) and an exit opening that are arranged to permit a pre-curved segment of a catheter (1, 34) to pass through the interior cavity (29),
the openings (19) are positioned to be off of the vertical center line (20) of the cross section of the balloon (14, 38, 39) taken at the openings (19) so that the balloon (14, 38, 39) is able to be affixed to a catheter (1, 34) in an off-center fashion such that, when the envelope is inflated, the balloon (14, 38, 39) provides a greater volume section of the Interior cavity (29) above the convex surface of an inserted pre-curved segment of such a catheter (1, 34) than the concave surface of the pre-curved segment.

2. The balloon (14, 38, 39) according to Claim 1 wherein the openings (19) are positioned on a line longitudinally circumscribing the outer surface of the envelope so as to form two line segments of differing lengths.

3. The balloon (14, 38, 39) according to Claim 1 wherein the tubular section (15) is a generally arched shape.

4. The balloon (14, 38, 39) according to Claim 3 wherein the generally arched shape is substantially the same as that of a pre-curved segment of a catheter (1, 34) that is to be inserted through the interior cavity (29).

5. A balloon angioplasty catheter (1, 34) comprising the balloon (14, 38, 39) of any preceding claim.

6. The balloon angioplasty catheter (1, 34) according to Claim 5, wherein the catheter comprises hollow tubing (8), attached at one end to a balloon (14, 38, 39) and attached at its other end to means for delivering sterile fluid (2, 3, 4, 5) through the tubing (8) and into the balloon (14, 38, 39) through said openings in the tubing (8), wherein the tubing (8) is operatively attached to the balloon (14, 38, 39) in the off-center fashion.

7. The balloon catheter (1) according to Claim 6 wherein the tubing (8) is attached to the balloon (14, 38, 39) at each of opposing end sections of the balloon (14, 38, 39).

8. The balloon catheter (1) according to Claim 6 comprising a further tubing (10, 36) within the tubing (8) and wherein a guide wire (9) extends through the further tubing (10, 36).

9. The balloon catheter (34) according to Claim 6 wherein the catheter (34) comprises:
a first balloon (38) fixed on one side of its central longitudinal axis (42) to permit the first balloon (38) to inflate first along the one side when sterile fluid is inserted into the first balloon (38), and
a second balloon (39) is fixed on one side of its central longitudinal axis (44) that is opposite the fixed side of the first balloon (38) to inflate first along the one side of the second balloon (39) when sterile fluid is inserted into the second balloon (39), the respective balloons (38, 39) being affixed to the catheter In the off-center fashion.

10. The balloon catheter (34) according to Claim 9 wherein the first balloon (38) is fixed on one side of its central longitudinal axis (42) to permit the first balloon (38) to inflate only along the one side of its central longitudinal axis (42) when sterile fluid is inserted into the first balloon (38).

11. The balloon catheter (34) according to Claim 9 wherein the second balloon (39) is fixed on one side of its longitudinal axis (44) to permit the second balloon (39) to inflate only along the one side of its central longitudinal axis (44) when sterile fluid is inserted into the second balloon (39).

12. The balloon catheter (1, 34) according to Claim 6 wherein a guide wire (9) extends through the hollow tubing (8).

13. The balloon catheter (1, 34) according to Claim 6 wherein the distal end section of the hollow tubing (8) is curved.

14. The balloon catheter (1, 34) according to any of Claims 5 to 13, wherein the hollow tubing (8) is operatively attached to the balloon (14, 38, 39) at a position off the central longitudinal axis (20) of the balloon (14, 38, 39).

## Patentansprüche

1. Ein Ballon (14, 38, 39), der zum Anbringen an einem Angioplastie-Katheter (1, 34) geeignet ist, der ein vorgekrümmtes Segment zum Positionieren eines Stents (26) oder zum Ausführen einer Angioplastie in einem gekrümmten Segment eines Gefäßes besitzt und ferner zum Aufblasen bzw. Aufweiten des Ballons (14,38,39) dient, wobei der Ballon (14,38,39) aufweist:
eine Hülle mit einem mittleren länglichen, allgemein schlauchförmigen, aufblasbaren Abschnitt (15) und zwei Endabschnitten (16, 17), wobei die zwei Endabschnitte (16, 17) so angeordnet sind, dass sie jeweilige geneigte Wandoberflächen besitzen, wenn der Ballon (14,38,39) an einem vorgekrümmten Segment eines Katheters (1,34) befestigt ist bzw. wird, wobei die Hülle einen inneren Hohlraum (29) definiert und die Hülle außerdem eine Aufnahmeöffnung (19) und eine Ausgangsöffnung besitzt, die angeordnet sind, damit ein vorgekrümmtes Segment eines Katheters (1,34) durch den inneren Hohlraum (29) hindurchtreten kann,
wobei die Öffnungen (19) von der vertikalen Mittellinie (20) des Querschnitts des Ballons (14,38,39) an den Öffnungen (19) versetzt angeordnet sind, derart, dass der Ballon (14,38,39) an einem Katheter (1,34) in einer exzentrischen Weise derart befestigt werden kann, dass, wenn die Hülleaufgeblasen bzw. aufgeweitet ist, der Ballon (14, 38, 39) einen größeren Volumenabschnitt des inneren Hohlraums (29) über der konvexen Oberfläche eines eingeführten vorgekrümmten Segments eines solchen Katheters (1,34) als der konkaven Oberfläche des vorgekrümmten Segments hat.

2. Der Ballon (14, 38, 39) gemäß Anspruch 1, wobei die Öffnungen (19) auf einer Linie angeordnet sind, die in Longitudinalrichtung die Außenoberfläche der Hülle so umschreibt, dass zwei Liniensegmente unterschiedlicher Längen gebildet werden.

3. Der Ballon (14, 38, 39) gemäß Anspruch 1, wobei der schlauchförmige Abschnitt (15) eine allgemein bogenartige Form besitzt.

4. Der Ballon (14, 38, 39) gemäß Anspruch 3, wobei die allgemein bogenartige Form im Wesentlichen die gleiche ist wie die eines vorgekrümmten Segments eines Katheters (1,34), der durch den inneren Hohlraum (29) einzuführen ist.

5. Ein Angioplastie-Ballonkauheter (1,34) mit dem Ballon (14,38,39) gemäß einem vorangehenden Anspruch.

6. Der Angioplastie-Ballonkatheter (1,34) gemäß Anspruch 5, wobei der Katheter eine hohle Rohrleitung (8) umfasst, die an einem Ende an einem Ballon (14,38,39) angebracht ist, und an ihrem anderen Ende an Mitteln zum Liefern von sterilem Fluid (2,3,4,5) durch die Rohrleitung (8) und in den Ballon (14,38,39) durch die Öffnungen in der Rohrleitung (8) angebracht ist, wobei die Rohrleitung (8) funktionsmäßig an dem Ballon (14,38,39) in der exzentrischen Weise angebracht ist.

7. Der Ballonkatheter (1) gemäß Anspruch 6, wobei die Rohrleitung (8) an dem Ballon (14,38,39) an jedem von entgegengesetzten Endabschnitten des Ballons (14, 38, 39) angebracht ist.

8. Der Ballonkatheter (1) gemäß Anspruch 6, mit einer weiteren Rohrleitung (10,36) in der Rohrleitung (8) und wobei ein Führungsdraht (9) sich durch die weitere Rohrleitung (10,36) erstreckt.

9. Der Ballonkatheter (34) gemäß Anspruch 6, wobei der Katheter (34) aufweist:
einen ersten Ballon (38), der an einer Seite seiner zentralen Longitudinalachse (42) befestigt ist, um ein Aufblasen bzw. Aufweiten des ersten Ballons (38) zuerst entlang der einen Seite zu ermöglichen, wenn steriles Fluid in den ersten Ballon (38) eingebracht wird, und
einen zweiten Ballon (39), der an einer Seite seiner zentralen Longitudinalachse (44), die der befestigten Seite des ersten Ballons (38) gegenüber liegt, befestigt ist, um zuerst entlang der einen Seite des zweiten Ballons (39) aufzuweiten, wenn steriles Fluid in den zweiten Ballon (39) eingebracht wird, wobei die jeweiligen Ballone (38,39) an dem Katheter in einer exzentrischen Weise angebracht sind.

10. Der Ballonkatheter (34) gemäß Anspruch 9, wobei der erste Ballon (38) an einer Seite seiner zentralen Longitudinalachse (42) befestigt ist, um ein Aufweiten des ersten Ballons (38) nur entlang der einen Seite seiner zentralen Longitudinalachse (42) zuzulassen, wenn steriles Fluid in den ersten Ballon (38) eingebracht wird.

11. Der Ballonkatheter (34) gemäß Anspruch 9, wobei der zweite Ballon (39) an einer Seite seiner Longitudinalachse (44) befestigt ist, um ein Aufweiten des zweiten Ballons (39) nur entlang der einen Seite seiner zentralen Longitudinalachse (44) zuzulassen, wenn steriles Fluid in den zweiten Ballon (39) eingebracht wird.

12. Der Ballonkatheter (1, 34) gemäß Anspruch 6, wobei ein Führungsdraht (9) sich durch die hohle Rohrleitung (8) erstreckt.

13. Der Ballonkatheter (1,34) gemäß Anspruch 6, wobei der distale Endabschnitt der hohlen Rohrleitung (8) gekrümmt ist.

14. Der Ballonkatheter (1,34) gemäß einem der Ansprüche 5 bis 13, wobei die hohle Rohrleitung (8) funktionsmäßig an dem Ballon (14,38,39) an einer Position versetzt von der zentralen Longitudinalachse (20) des Ballons (14,38,39) angebracht ist.

## Revendications

1. Ballonnet (14, 38, 39) adapté pour être apposé sur un cathéter d'angioplastie (1, 34) ayant un segment pré-courbé permettant de positionner une endoprothèse vasculaire (26) ou de réaliser une angioplastie dans un segment courbé d'un vaisseau et en outre pour gonfler le ballonnet (14, 38, 39), dans lequel le ballonnet (14, 38, 39) comprend :
une enveloppe ayant une section médiane gonflable, généralement tubulaire et allongée (15) et deux sections d'extrémité (16, 17), les deux sections d'extrémité (16, 17) étant agencées pour comporter des surfaces de paroi inclinées respectives lorsque le ballonnet (14, 38, 39) est apposé sur un segment pré-courbé d'un cathéter (1, 34), l'enveloppe définissant une cavité intérieure (29), l'enveloppe comprenant également une ouverture de réception (19) et une ouverture de sortie qui sont agencées pour permettre à un segment pré-courbé d'un cathéter (1, 34) de passer à travers la cavité intérieure (29),
les ouvertures (19) sont positionnées pour être décalées de la ligne centrale verticale (20) de la section du ballonnet (14, 38, 39) prise aux ouvertures (19) de sorte que le ballonnet (14, 38, 39) peut être apposé sur un cathéter (1, 34) de façon excentrée de sorte que, lorsque que l'enveloppe est gonflée, le ballonnet (14, 38, 39) fournit une section de volume plus grande de la cavité intérieure (29) au-dessus de la surface convexe d'un segment pré-courbé inséré d'un tel cathéter (1, 34) que la surface concave du segment pré-courbé.

2. Ballonnet (14, 38, 39) selon la revendication 1 dans lequel les ouvertures (19) sont positionnées sur une ligne circonscrivant longitudinalement la surface externe de l'enveloppe de façon à former deux segments de ligne de longueurs différentes.

3. Ballonnet (14, 38, 39) selon la revendication 1, dans lequel la section tubulaire (15) est une forme généralement arquée.

4. Ballonnet (14, 38, 39) selon la revendication 3, dans lequel la forme généralement arquée est sensiblement la même que celle d'un segment pré-courbé d'un cathéter (1, 34) qui doit être inséré à travers la cavité intérieure (29).

5. Cathéter d'angioplastie à ballonnet (1, 34) comprenant le ballonnet (14, 38, 39) selon l'une quelconque des revendications précédentes.

6. Cathéter d'angioplastie à ballonnet (1, 34) selon la revendication 5, dans lequel le cathéter comprend une tubulure creuse (8) attachée à une extrémité à un ballonnet (14, 38, 39) et attachée à son autre extrémité à des moyens permettant de délivrer un fluide stérile (2, 3, 4, 5) à travers la tubulure (8) et dans le ballonnet (14, 38, 39) à travers lesdites ouvertures dans la tubulure (8), dans lequel la tubulure (8) est attachée de manière opérationnelle au ballonnet (14, 38, 39) de façon excentrée.

7. Cathéter à ballonnet (1) selon la revendication 6 dans lequel la tubulure (8) est attachée au ballonnet (14, 38, 39) à chacune des sections d'extrémité opposées du ballonnet (14, 38, 39).

8. Cathéter à ballonnet selon la revendication 6 comprenant une tubulure supplémentaire (10, 36) à l'intérieur de la tubulure (8) et dans lequel un fil guide (9) s'étend à travers la tubulure supplémentaire (10, 36).

9. Cathéter à ballonnet (34) selon la revendication 6 dans lequel le cathéter (34) comprend :
un premier ballonnet (38) fixé sur un côté de son axe longitudinal central (42) afin de permettre au premier ballonnet (38) de se gonfler en premier lieu le long dudit côté lorsque du fluide stérile est inséré dans le premier ballonnet (38), et
un second ballonnet (39) est fixé sur un côté de son axe longitudinal central (44) qui est opposé au côté fixe du premier ballonnet (38) afin de se gonfler en premier lieu le long dudit côté du second ballonnet (39) lorsque du fluide stérile est inséré dans le second ballonnet (38), les ballonnets respectifs (38, 39) étant apposés sur le cathéter de manière excentrée.

10. Cathéter à ballonnet (34) selon la revendication 9 dans lequel le premier ballonnet (38) est fixé sur un côté de son axe longitudinal central (42) afin de permettre au premier ballonnet (38) de se gonfler uniquement le long dudit côté de son axe longitudinal central (42) lorsque du fluide stérile est inséré dans le premier ballonnet (38).

11. Cathéter à ballonnet (34) selon la revendication 9 dans lequel le second ballonnet (39) est fixé sur un côté de son axe longitudinal (44) afin de permettre au second ballonnet (39) de se gonfler uniquement le long dudit côté de son axe longitudinal central (42) lorsque du fluide stérile est inséré dans le second ballonnet (39).

12. Cathéter à ballonnet (1, 34) selon la revendication 6 dans lequel un fil guide (9) s'étend à travers la tubulure creuse (8).

13. Cathéter à ballonnet (1, 34) selon la revendication 6 dans lequel la section d'extrémité distale de la tubulure creuse (8) est courbée.

14. Cathéter à ballonnet (1, 34) selon l'une quelconque des revendications 5 à 13, dans lequel la tubulure creuse (8) est attachée de manière opérationnelle au ballonnet (14, 38, 39) à une position décalée par rapport à l'axe longitudinal central (20) du ballonnet (14, 38, 39).
